(19) Europäisches Patentamt
European Patent Office
Office européen des brevets

(11) EP 2 048 133 A1

(12) EUROPEAN PATENT APPLICATION

(43) Date of publication:
**15.04.2009 Bulletin 2009/16**

(51) Int Cl.:
***C07D 213/89*** (2006.01) ***C07D 401/04*** (2006.01)
***A61K 31/4709*** (2006.01) ***A61P 29/00*** (2006.01)

(21) Application number: **07019421.2**

(22) Date of filing: **04.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Pharmeste S.r.l.**
**44100 Ferrara (IT)**

(72) Inventors:
- **Napoletano, Mauro c/o Pharmeste S.r.l**
  **44100 Ferrara (IT)**
- **Pavani, Maria Giovanna**
  **44100 Ferrara (IT)**
- **Fruttarolo, Francesca c/o Pharmeste S.r.l**
  **44100 Ferrara (IT)**
- **Trevisani, Marcello**
  **44100 Ferrara (IT)**

(74) Representative: **Minoja, Fabrizio**
**Bianchetti Bracco Minoja S.r.l.**
**Via Plinio, 63**
**20129 Milano (IT)**

# (54) Nicotinamide n-oxide derivatives as vanilloid-1 receptor modulators

(57) The present invention relates to vanilloid receptor modulators of formula (I)

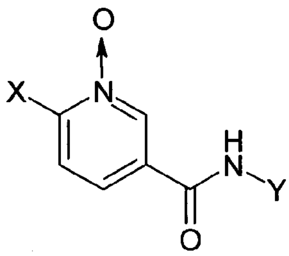

**(I)**

wherein X is selected from phenyl, naphthyl, pyridine, quinoline or isoquinoline and Y is optionally substituted phenyl or pyridyl ring, to processes for the preparation thereof and to their pharmaceutical uses for the treatment of inflammatory states.

EP 2 048 133 A1

## Description

### Field of the invention

The present invention relates to modulators of the vanilloid receptor, in particular to TRPV1 antagonists.

### State of the art

The Transient Receptor Potential Vanilloid 1 is strongly involved in the genesis of different pathological conditions including neuropathic pain and urological disorders.

Vanilloid receptor antagonists containing an acetamido moiety, more specifically a nicotinamide moiety, have been disclosed, for example, in US 2005/085512, US 2005/165028, WO 2005/032493, WO 2005/034870, WO 2005/030753, W02003/068749.

### Description of the invention

The present invention relates to nicotinamide N-oxide derivatives that are able to modulate, especially to antagonize, the transient receptor potential vanilloid 1 receptor.

The compounds of the invention have general formula (I)

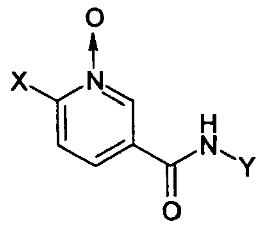

**(I)**

wherein:

X is selected from phenyl, naphthyl, pyridine, quinoline or isoquinoline;
Y is a phenyl or pyridyl ring, optionally substituted with one or two R groups independently selected from $C_1$-$C_4$ alkyl, $C_1$-$C_4$ haloalkyl, or halogen.

The expression $C_1$-$C_4$ alkyl indicates a methyl, ethyl, propyl, isopropyl, sec-butyl or a *tert*-butyl group; preferably a methyl, isopropyl or *tert*-butyl group.

The expression $C_1$-$C_4$ haloalkyl indicates a methyl, ethyl, propyl, isopropyl, sec-butyl or a *tert*-butyl group substituted with one or more halogen atoms selected from fluorine, chlorine, bromine or iodine; a preferred $C_1$-$C_4$ haloalkyl group is the trifluoromethyl group.

The term halogen indicates a halogen atom selected from fluorine, chlorine, bromine and iodine, preferably chlorine.

Preferred compounds of the invention are those wherein X is an isoquinolinyl group, in particular an isoquinolin-5-yl group, and Y is an optionally substituted phenyl, i.e. compounds of formula **(IA)**:

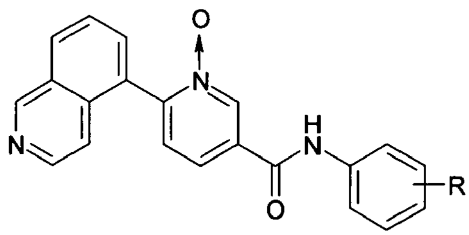

**(IA)**

wherein R is as defined above.

A particularly preferred compound of the invention is the compound of formula **(IA)** wherein R is a chlorine atom at position 4, i.e. the compound of formula 1 (in the following also referred to as V405)

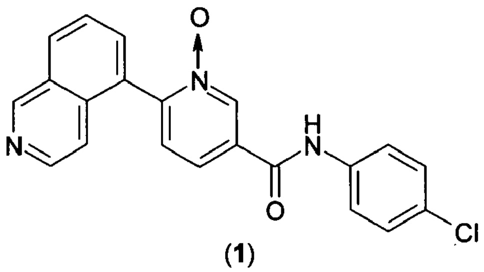

**(1)**

With respect to compounds wherein the pyridine ring is not oxidized, the compounds of the invention are remarkably more soluble, still retaining the *in vitro* affinity and the *in vivo* activity towards the TRPV1 receptor. For example, the thermodynamic solubility of compound **1** in PBS is 106 μM, while its non-oxidized homologous is not soluble.

Other advantages of N-oxide derivatives in comparison with the simple nicotinamide compounds are in terms of safety. Unlike its non-oxidized homologous, compound **1** does not interact with a panel of cytochromes (therefore, it does not interfere with the metabolism of other drugs) and does not cause thermal increase.

The compounds of formula **(I)** can be prepared by means of conventional methods, for example by Suzuki reaction of a compound of formula (**II**)

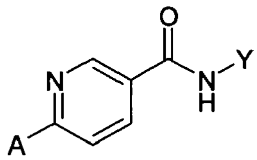

**(II)**

wherein Y is as defined above and A is a halogen atom selected from iodine, bromine and chlorine with a boronic acid of formula **(III)**

$X\text{-}B(OH)_2$ **(III),**

followed by oxidation of the pyridine nitrogen with a suitable oxidizing agent, for example with metachloroperbenzoic acid or acetic acid/hydrogen peroxide.

The compounds of the invention modulate the vanilloid TRPV1 receptor and can be used for the preparation of pharmaceutical compositions for the treatment of inflammatory states, such as chronic pain, inflammatory hyperalgesia, neuropathic pain and overactive bladder. These compositions can be prepared by means of conventional methods and excipients, such as those disclosed in Remington's Pharmaceutical Science Handbook, XIX ed. Mack Pub., N.Y., U.S.A.

The invention will be herein after illustrated in greater detail in the following experimental section.

## EXAMPLES

## CHEMISTRY

### Materials and methods

All commercially available compounds were purchased from Aldrich and were used without further purification. Reaction courses were monitored by thin-layer chromatography on silica gel (precoated $F_{254}$ Merck plates), the spots were examined with UV light and visualized with aqueous $KMnO_4$ Flash chromatography was performed using Merck silica gel (230-240 mesh). $^1H$-NMR spectra were recorded on a Varian 400 MHz spectrometer using TMS as internal standard. Mass spectra were obtained with a Waters-Micromass ZMD spectrometer. Melting points were determined

on a Buchi-Tottoli apparatus and are uncorrected.

**EXAMPLE 1 - Synthesis of N-(4-chlorophenyl)-6-(isoquinolin-5-yl)pyridine-3-carboxamide N-oxide (compound I, or V405)**

**Step 1: Synthesis of 6-chloro-N-(4-chlorophenyl)pyridine-3-carboxamide**

Commercially available 6-chloronicotinic chloride (56,8 mmol, 10 g) was dissolved in 50 ml of anhydrous $CH_2Cl_2$ and added dropwise to a solution of DIEA (1.2 equivalents, 68.2 mmol, 11.67 ml) and 4-chloroaniline (1.2 equiv., 68.2 mmol, 8.70 g) in 50 ml $CH_2Cl_2$ at 0°C. The reaction was stirred at room temperature for 20 h. The mixture was then diluted with $CH_2Cl_2$ (200 ml) and washed with water (1 x 200 ml) and Brine (1 x 100 ml). The organic layer was dried over sodium sulphate and concentrated. The crude residue was crystallized from diethyl ether to give 13 g of a white solid.

Yield = 86%. $^1H$ NMR ($CDCl_3$, 200 MHz) δ 7.35 (2H, d, J= 8.8 Hz), 7.47 (1 H, d, J= 8.2 Hz), 7.58 (2H, d, J= 8.8 Hz), 7.88 (1H, bs), 8.16 (1H, dd, J= 8.4 Hz, J'= 2.8 Hz), 8.84 (1 H, d, J= 2.4Hz).

**Step 2: Suzuki reaction for the preparation of N-(4-chlorophenyl)-6-(isoquinolin-5-yl)pyridine-3-carboxamide**

A mixture of isoquinolin-5-yl-5-boronic acid (1.5 equiv., 8.4 mmol, 1.46 g, prepared as described below), 6-chloro-N-(4-chlorophenyl)pyridine-3-carboxamide (5.6 mmol, 1.5 g), palladium acetate (4% mol, 48 mg), triphenylphosphine (2 equiv., 2,94 g), 15% $Na_2CO_3$ (4 ml), EtOH (4 ml) and toluene (50 ml) was heated at 80°C for 16 h. After evaporation, a saturated sodium bicarbonate solution was added and the solid was filtered and washed with ethyl acetate. The crude residue was recrystallized from methanol to obtain 1.4 g of a white solid. M.p. (diethyl ether) = 258°C. Yield = 69%. $^1H$ NMR ($d_6$-DMSO, 400 MHz) δ 7.471 (2H, d, J= 8.8 Hz), 7.838 (1H, m), 7.852 (2H, d, J= 8.8 Hz), 7.953 (1 H, d, J= 8.0 Hz), 8.049 (1 H, dd, J= 7.2 Hz, J'= 0.8 Hz), 8.085 (1H, d, J= 6.0 Hz), 8.290 (1H, d, J= 8.4 Hz), 8.490 (1 H, dd, J= 8.4 Hz, J'= 2.2 Hz), 8.543 (1 H, d, J= 6.0 Hz), 9.300 (1 H, d, J= 1.6 Hz), 9.438 (1H, s), 10.695 (1H, s); [$M^{+1}$] 360.4 ($C_{21}H_{14}ClN_3O$ requires 359.81).

**Step 3: Oxidation reaction for the preparation of compound V405**

Trifluoromethanesulfonic acid (1.5 equiv., 180 μl) was added to a solution of N-(4-chlorophenyl)-6-(isoquinolin-5-yl)pyridine-3-carboxamide (1 g, 3.76 mmol) in 50 ml of glacial acetic acid and precipitation of a white solid was observed. 30% $H_2O_2$ (10 equivalents, 5 ml) was then added and the mixture was heated at 80°C overnight. After cooling to room temperature, 10% Pd/C was added to destroy any peroxide excess. The mixture was filtered over Celite and washed with toluene. The filtrate was evaporated to dryness and the crude residue was purified by chromatographic column (AcOEt 9 / MeOH 1 followed by AcOEt 8 / MeOH 2) to give 450 mg of a white solid corresponding to V405. Yield = 32%. $^1H$ NMR δ ($d_6$-DMSO, 200 MHz): 7.30 (1H, d, J= 5.8 Hz), 7.49 (2H, d, J= 8.8 Hz), 7.83 (3H, m), 7.85 (2H, d, J= 8.8 Hz), 7.95 (1 H, dd, J= 8.0 Hz, J'= 1.6 Hz), 8.38 (1 H, dd, J= 7.0 Hz, J'= 2.0 Hz), 8.49 (1H, d, J= 5.8 Hz), 8.97 (1H, d, J= 1.8 Hz), 9.43 (1H, s), 10.69 (1 H, s).

**Synthesis of isoquinolin-5-yl-5-boronic acid**

A 2.5 M solution of n-BuLi (1.2 equiv., 3 mmol, 1.2 ml) in 20 ml of freshly distilled THF, cooled to -78°C was added with a solution of 5-bromoisoquinoline (2.5 mmol, 520 mg) in 5 ml of THF. The resulting mixture was allowed to react at this temperature over 45'. A solution of triisopropylborate (1.2 equiv., 3 mmol, 0.7 ml) was then added and the mixture was stirred at the same temperature for 5' and then allowed to warm to room temperature and stirred for an additional hour. The mixture was quenched by slow addition of a 5% NaOH solution (30 ml). The aqueous layer was separated and acidified to pH 5/6 by addition of HCl 10% at O°C. Extraction with ethyl acetate, evaporation of the organic phase and crystallisation from diethyl ether gave 250 mg of a white solid. Yield = 58%. $^1H$ NMR ($d_6$-DMSO, 200 MHz) δ 7.66 (1H, t, J= 7.2 Hz), 8.07 (1H, d, J= 5.8 Hz), 8.13 (1H, d, J= 8.0 Hz), 8.32 (1 H, d, J= 6.2 Hz), 8.47 (1H, d), 8.50 (2H, bs), 9.29 (1 H, s); [$M^{+1}$] 174.1 ($C_9H_8BNO_2$ requires 172.98).

**Example 2 - Synthesis of N-(4-chlorophenyl)-6-(naphthalen-8-yl)pyridine-3-carboxamide N-oxide**

**Step 1: Synthesis of N-(4-chlorophenyl)-6-(naphthalen-8-yl)pyridine-3-carboxamide**

A mixture of naphthalen-1-yl-boronic acid (1.5 equiv., 8.4 mmol, 1.44 g), 6-chloro-N-(4-chlorophenyl)pyridine-3-carboxamide (5.6 mmol, 1.5 g), palladium acetate (4% mol, 48 mg), triphenylphosphine (2 equiv., 2,94 g), 15% $Na_2CO_3$ (4 ml), EtOH (4 ml) and toluene (50 ml) was heated at 80°C for 16 h. After evaporation, a saturated sodium bicarbonate

solution was added and,the solid was filtered and washed with ethyl acetate. The crude residue was recrystallized from methanol to obtain 1.6 g of a white solid. M.p. (diethyl ether) = 277°C. Yield = 79%. $^1$H NMR ($CDCl_3$, 400 MHz) δ 7.339 (2H, d, J= 8.8 Hz), 7.544 (4H, m), 7.666 (2H, d, J= 6.8 Hz), 7.738 (1 H, d, J= 8.0 Hz), 7.919 (2H, d, J= 8.0 Hz), 8.005 (1 H, d, J= 8.4 Hz), 8.354 (2H, m), 9.351 (1 H, s).

**Step 2: Synthesis of compound N-(4-chlorophenyl)-6-(naphthalen-8-yl)pyridine-3-carboxamide N-oxide**

Metachloroperbenzoic acid (1.2 equiv., 350 mg) was added to a solution of N-(4-chlorophenyl)-6-(isoquinolin-5-yl)pyridine-3-carboxamide (500 mg, 1.88 mmol) in 30 ml of chloroform. The mixture was stirred at room temperature overnight, diluted and washed with a saturated sodium bicarbonate solution. Evaporation of the organic phase and crystallisation from diethyl ether gave 500 mg of a white solid. M.p. (diethyl ether) = 211-212°C. Yield = 71%. $^1$H NMR ($d_6$-DMSO, 400 MHz) δ 7.389 (1H, d, J= 8.0Hz), 7.481 (3H, m), 7.595 (2H, m), 7.654 (1H, m), 7.799 (1H, d, J= 8.0 Hz), 7.850 (2H, m), 7.948 (1 H, dd, J= 8.0 Hz, J'= 2.0 Hz), 8.051 (1 H, d, J= 8.0 Hz), 8.111 (1 H, d, J= 8.4 Hz), 8.944 (1 H, d, J= 1.2 Hz), 10.689 (1 H, s).

**THERMODYNAMIC SOLUBILITY OF V405 IN PBS**

The standard solution was prepared by dissolving 1.4 mg of V405 in 10 mL of acetonitrile. A saturated solution of V405 was prepared by stirring 10 mL of phosphate buffer pH 7.4 in the presence of 10 mg of compound. The stirring was conducted at room temperature for 36h. The supernatant was filtered through a Millipore 0.45mm filter and 10 μL were used as sample for the HPLC determination. (Eluents: A = Water with 0.1% TFA, B = Acetonitrile + 0.1% TFA, Gradient: 0-100% B in 25 min, Column: SYMMETRY 300 C18 5 μM; 4,6 X 150 MM, Injection Volume: 15 μL). The solubility was determined by the following relationship: C'= percent area' C/percent area where C= concentration of standard solution (mg/ml), percent area': area of the peak of standard solution, percent area: area of the peak of saturated solution, C'= concentration of the saturated solution. The solubility was then expressed as polarity.

**INHIBITION OF CYTOCROME P450 SUBTYPES BY V405**

A microtiter plate-based, direct, fluorometric assay was performed to analyze V405 inhibition potential of activities catalyzed by six of the major human liver cytochromes P450 (CYP) enzymes: CYP1A2, CYP2C9, CYP2C19, CYP2D6, CYP2E1 and CYP3A4. The six cytochrome P450 subtypes inhibition was tested from 0.1 μM to10 μM.

Four dilution points were evaluated (10 μM, 2.15 μM, 0.46 μM and 0.1 μM). A lid was placed on the plate and plate was placed at 37°C in a plate incubator for at least 10 minutes (to pre-warm the buffer and the plate). The reaction started when 0.1 mL enzyme/substrate solution mix was added to test wells. The plate was mixed and the reaction was continued incubating the lid-covered plate at 37°C for the desired time.

A stop solution was then added to all wells. and an enzyme/substrate mix was dispensed to the inverted blank wells (negative controls).

>The $IC_{50}$ was calculated with a typical logistic curve fitting :

$$\% \text{ Inib} = \frac{\text{Min} - \text{Max}}{1 + \left( \frac{\text{Conc}}{IC_{50}} \right)^p} + \text{Max}$$

**PHARMACOLOGY**

**Radioligand binding assay**

Male Sprague-Dawley rats with body weight between 250 to 350 g at the time for testing were used. For binding assays rats were sacrificed by decapitation under anesthesia and the spinal cord was removed and disrupted using a Polytron tissue homogenizer in ice cold buffer containing 5 mM KCl, 5.8 mM NaCl, 0.75 mM $CaCl_2$, 2 mM $MgCl_2$, 320 mM sucrose, 10 mM Hepes, pH 8.6.[5] The homogenized tissue was centrifuged at 1000 x g for 10 min at 4°C and the supernatant was centrifuged again at 35000 x g for 30 min at 4°C (Beckman Avanti J25). The pellet was resuspended in the same buffer as described above and used in binding experiments. In saturation experiments, 150 μg protein/sample from membrane suspensions were incubated with [$^3$H]-Resiniferatoxin ([$^3$H]-RTX) (0.003-3 nM) in the assay

buffer containing 0.25 mg/ml fatty acid-free bovine serum albumin at 37°C for 60 min. In competition experiments, the membranes were incubated at 37°C for 60 min with [$^3$H]RTX (0.4 nM) and with increasing concentrations (from 0.1 nM to 3 μM) of examined compounds.

Non specific binding was evaluated in the presence of 1 μM RTX. After incubation the reaction mixture was cooled at 0°C and incubated with bovine α1-acid glycoprotein (200 μg per tube) for 15 min to reduce non-specific RTX binding. Membrane-bound RTX was separated from free RTX by centrifuging the samples at 18500 x g for 15 min. The tip of the microcentrifuge tube containing the pellet was cut off and the radioactivity was determined by scintillation counting (Packard 2500 TR). Protein concentration was determined according to a Bio-Rad method with bovine serum albumin as a standard reference (Bradford, 1976). Saturation and competition studies were analyzed with the Ligand program.

**$Ca^{2+}$ fluorescence measurements in cultured rat dorsal root ganglia neurones**

Adult rats were terminally anaesthetized and decapitated. Dorsal root ganglia were removed and placed in cold phosphate buffered solution (PBS) before being transferred to collagenase (10 mg/ml), trypsin (5 mg/ml) and DNAse (1 mg/ml) for 35 min at 37°C. The ganglia, placed in cold DMEM supplemented with 10% fetal bovine serum, 2 mM L-glutamine, 100 U/ml penicillin and 100 μg/ml streptomycin, were dissociated in single cells by several passages through a series of syringe needles (23G down to 25G). The medium and the ganglia were filtered to remove debris, topped up with 8 ml of DMEM medium and centrifuged (200 x g for 5 min). The final cell pellet was re-suspended in DMEM medium [supplemented with 100 ng/ml mouse Nerve Growth Factor (mouse-NGF-7S) and cytosine-β-D-arabinofuranoside free base (ARA-C) 2.5 μM]. The cells were plated on poly-L-lysine (8.3 μM)- and laminin (5 μM)- coated 25 mm glass cover slips and kept for 5 to 8 days at 37°C in a humidified incubator gassed with 5% $CO_2$ and air, then added with Fura-2-AM-ester (3 μM) in a $Ca^{2+}$ buffer solution having the following composition (mM): $CaCl_2$ 1.4, KCl 5.4, $MgSO_4$ 0.4, NaCl 135, D-glucose 5, HEPES 10 with BSA (0.1%), at pH 7.4, for 40 min at 37°C. The cells were then washed twice with the $Ca^{2+}$ buffer solution and transferred to a chamber on the stage of a Nikon eclipse TE300 microscope. Fura-2-AM-ester was excited at 340 nM and 380 nM to indicate relative $[Ca^{2+}]_i$ changes by the $F_{340}/F_{380}$ ratio recorded with a dynamic image analysis system (Laboratory Automation 2.0, RCS, Florence, Italy) and the cells were allowed (at least 10 min) to attain a stable fluorescence before beginning the experiment. A calibration curve was set up using buffer containing Fura-2-AM-ester and determinant concentrations of free $Ca^{2+}$. This curve was then used to convert the data obtained from the $F_{340}/F_{380}$ ratio to $[Ca^{2+}]_i$ (nM). The effects of pretreatments with capsazepine (CPZ), SB366791 and V405 on the increase in $[Ca^{2+}]_i$ produced by 0.1 μM capsaicin were studied.

**Capsaicin-induced secondary allodynia in rat**

Capsaicin (5 nmols/50 μl/paw) was injected in the plantar surface of the glabrous skin of the right paw of rats anesthetized with diethyl ether (Chaplan et al., 1994). Compound V405 was orally administrated (30 μmol/kg) 2 hours prior to capsaicin injection. Tactile allodynia was evaluated 30 min after capsaicin challenge.

**Body Temperature Measurements**

Rats were placed in individual cages at room temperature (22-25°C). Rectal temperatures were recorded from awake animals by repeated insertion of a lubricated rectal thermocouple probe (model-8402-00, Cole-Palmer Instrument Company) prior to and following orally administration of V405 at 30 μmil/kg.

**Drugs and Reagents**

Drugs and reagents were obtained from the indicated companies: [$^3$H]-Resiniferatoxin (Perkin Elmer, Boston, MA), SB-366791 (Tocris, UK), capsaicin, capsazepine, ionomycin, laminin, poly-L-lysine, substance P (Sigma, Italy); mice NGF-7S and collagenase/dispase (Roche Diagnostics, Italy); Dulbecco's Modified Eagle's medium (DMEM), foetal bovine serum (FBS) heat inactivated, L-glutamine (200 mM), penicillin/streptomycin (10,000 IU/ml ± 10,000 UG/ml), (Gibco, Italy); Fura-2-AM-ester (Società Italiana Chimici, Italy). The stock concentrations of capsaicin (10 mM), capsazepine (10 mM), (*E*)-3-(4-chlorophenyl)-N-(3-methoxyphenyl)acrylamide (identified as SB-366791) (1 mM) and V405 were prepared in 50% DMSO and 50% Tween 80. Fura-2-AM-ester and ionomycin were dissolved in 100% DMSO. All other drugs were dissolved in distilled water. Appropriate dilutions were then prepared in Krebs buffer solution.

## Results

### Thermodynamic solubility of V405 in PBS

Thermodynamic solubility of compound V405 under the described conditions was calculated as 106.4 μM whereas, in the same conditions the homologous nicotinamide derivative is not soluble.

### Inhibition of cytochromes P450 subtypes by V405

Compound V450 does not interact with any of the evaluated cytochromes at each concentration tested.

Table: **Cytochrome P450 subtypes μM inhibition (estimated $IC_{50}$)**

| | CYP1A2 | CYP2C9 | CYP2C19 | CYP2D6 | CYP2E1 | CYP3A4 |
|---|---|---|---|---|---|---|
| **V405** | >10 μM | >10 μM | >10 μM | >10 μM | >10 μM | >10 μM |

### Radioligand Binding Assay

The saturation curve of [$^3$H]-RTX to TRPV1 expressed in rat spinal cord showed a $K_D$ value of 0.21 (0.16-0.27) and a $B_{max}$ value of 57 (53-62) fmol/mg protein. The Scatchard plot was essentially linear and computer analysis of the data indicated that only one class of high affinity binding sites was present. Competition binding experiments of [$^3$H]-RTX revealed that V405 and the reference SB-366791 had a $K_i$ value of 203 (167-246) nM and 36 (30-43) nM respectively.

### $Ca^{2+}$ fluorescence

Capsaicin (0.1 μM) caused an increase in [$Ca^{2+}$] in the vast majority (95%) of dorsal root ganglia neurons, which were thereby identified as TRPV1 expressing neurons. The $IC_{50}$ value of V405 that inhibited capsaicin-evoked $[Ca^{2+}]_i$ mobilization was 15 (10-22) nM. The reference TRPV1 antagonists, capsazepine and SB-366791, inhibited the capsaicin response with an $IC_{50}$ of 948 (676-1330) nM and 8.7 (3.4-17.3) nM, respectively. The results are expressed as mean and 95% fiducial limits.

### Capsaicin-induced secondary allodynia in rat

30 min. after the capsaicin challenge, compound V405 produced a significant preventive effect (55%) against the pro-allodinic effect of capsaicin.

### Body Temperature Measurements

It has been shown that some TRPV1 antagonists (Amgen, Gavva NR 2005) induce an increase in body temperature, most likely interacting with the temperature-dependent gating of the channel. To this purpose, the non oxidized homologous and V405 were tested on body temperature variations in rats, at the dose of 30 μmol/kg (orally). Temperature c hanges were monitored for more than 3 hours. No significant temperature change was observed in animals treated with compound V405. In contrast, its nicotinamide homologous induced a significant temperature increase (a mean value of +0.5°C) with respect to control animals during the whole experiment.

## Claims

**1.** A compound of formula (I)

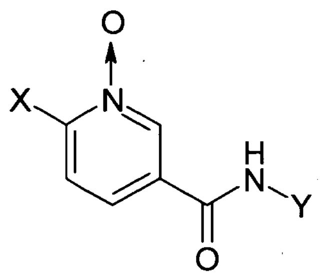

(I)

wherein:

X is selected from phenyl, naphthyl, pyridine, quinoline or isoquinoline;
Y is a phenyl or pyridyl ring, optionally substituted with one or two R groups independently selected from $C_1$-$C_4$ alkyl, $C_1$-$C_4$ haloalkyl, or halogen.

2. A compound according to claim 1 wherein X is isoquinolin-5-yl and Y is phenyl, optionally substituted with an R group as defined in claim 1.

3. A compound according to claim 2, wherein X is isoquinolin-5-yl and Y is 4-chlorophenyl.

4. A compound of any one of claims 1-3 for use as medicament.

5. The use of a compound of any one of claims 1-3 for the preparation of a pharmaceutical composition for the treatment of inflammatory states.

6. The use according to claim 5 wherein the inflammatory state is selected from chronic pain, inflammatory hyperalgesia, neuropathic pain and overactive bladder.

7. A pharmaceutical composition containing a compound of any one of claims 1-3 in admixture with suitable carriers and/or excipients.

European Patent Office

# EUROPEAN SEARCH REPORT

Application Number
EP 07 01 9421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2004/056774 A (NEUROGEN CORP [US]; BAKTHAVATCHALAM RAJAGOPAL [US]; BLUM CHARLES A [US) 8 July 2004 (2004-07-08)<br>* abstract *<br>* compounds 3,5,8-11,41-188, 190-213, 215-331, 332-389,391, *<br>* compounds 435-454, 459-473 *<br>* examples 4-10 *<br>* claims 1-119 *<br>----- | 1-7 | INV.<br>C07D213/89<br>C07D401/04<br>A61K31/4709<br>A61P29/00 |
| Y | MCDONNELL, MARK E. ET AL: "Efforts to circumvent major metabolic pathways of oxidation and glucuronidation in arylurea-based vanilloid ( TRPV1 ) series"<br>ABSTRACTS OF PAPERS, 229TH ACS NATIONAL MEETING, SAN DIEGO, CA, UNITED STATES, MARCH 13-17, 2005 , MEDI-102 PUBLISHER: AMERICAN CHEMICAL SOCIETY, WASHINGTON, D. C. CODEN: 69GQMP, 2005, XP009096394<br>* abstract *<br>----- | 1-7 | |
| | | | TECHNICAL FIELDS SEARCHED (IPC) |
| Y | WO 2005/040100 A (BAYER HEALTHCARE AG [DE]; BOUCHON AXEL [DE]; DIEDRICHS NICOLE [DE]; HE) 6 May 2005 (2005-05-06)<br>* abstract; claims 1-22; examples 3-16 *<br>* page 124 - page 127; tables 1-4 *<br>----- | 1-7 | C07D<br>A61K<br>A61P |
| Y | WO 02/053544 A (DARWIN DISCOVERY LTD [GB]; CUTSHALL NEIL S [US]; YAGER KRAIG M [US]) 11 July 2002 (2002-07-11)<br>* abstract; examples 2,3,5,7; table 3 *<br>* page 7, line 1 - line 7 *<br>* claims 1-44 *<br>-----<br>-/-- | 1-7 | |

The present search report has been drawn up for all claims

| Place of search | Date of completion of the search | Examiner |
|---|---|---|
| Munich | 22 February 2008 | Papathoma, Sofia |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 01 9421

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2008/006480 A (PHARMESTE S R L [IT]; BARALDI PIER GIOVANNI [IT]; BOREA PIER ANDREA [I) 17 January 2008 (2008-01-17)<br>* the whole document *<br>in particular example (compound V394) and claims 4 and 5<br>----- | 1-7 | |
| E | EP 1 889 830 A (PHARMESTE S R L [IT]) 20 February 2008 (2008-02-20)<br>* the whole document *<br>in particular scheme 3, claim 5 and 2nd and last compound of claim 6<br>----- | 1-7 | |

TECHNICAL FIELDS SEARCHED (IPC)

The present search report has been drawn up for all claims

2

| Place of search | Date of completion of the search | Examiner |
|---|---|---|
| Munich | 22 February 2008 | Papathoma, Sofia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 07 01 9421

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2008

| Patent document cited in search report | Publication date | Patent family member(s) | Publication date |
|---|---|---|---|
| WO 2004056774 A | 08-07-2004 | AU 2003299797 A1 | 14-07-2004 |
| | | CA 2510471 A1 | 08-07-2004 |
| | | EP 1575918 A2 | 21-09-2005 |
| WO 2005040100 A | 06-05-2005 | CA 2542494 A1 | 06-05-2005 |
| | | JP 2007509846 T | 19-04-2007 |
| WO 02053544 A | 11-07-2002 | NONE | |
| WO 2008006480 A | 17-01-2008 | EP 1889830 A1 | 20-02-2008 |
| EP 1889830 A | 20-02-2008 | WO 2008006480 A1 | 17-01-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2005085512 A
- US 2005165028 A
- WO 2005032493 A
- WO 2005034870 A
- WO 2005030753 A
- WO 2003068749 A


### Non-patent literature cited in the description


- Remington's Pharmaceutical Science Handbook. Mack Pub,